# EUROPEAN PATENT SPECIFICATION

(11) **EP 0 524 150 B1**
(45) Date of publication and mention of the grant of the patent: **17.11.1999**
(21) Application number: 92810529.5
(22) Date of filing: 10.07.1992
(51) Int. Cl.: A61L 2/18, A01N 25/30, G02C 13/00, A01N 59/00

(54) **Improvement in hydrogen peroxide disinfection solutions**
Verbesserung von Wasserstoffperoxid-Desinfektionslösungen
Amélioration de solutions désinfectrices de peroxyde d'hydrogène

(30) Priority: 19.07.1991 US 733115
(43) Date of publication of application: 20.01.1993
(73) Proprietor: Novartis AG, 4058 Basel (CH)
(72) Inventor: Winterton, Lynn C., Roswell, GA 30076 (US); Su, Kai C., Alpharetta, GA 30201 (US)

(56) References cited:
- EP-A- 0 441 389
- DE-A- 3 626 082
- US-A- 4 414 127

## Description

The present invention provides an improvement in hydrogen peroxide disinfection solutions. Specifically, the invention provides improvement in such disinfection solutions employed in the disinfection of contact lenses.

Disinfecting solutions for use with contact lenses are well known in the art and the use of such lenses involves a daily disinfecting treatment. Flexible, or soft, contact lenses are generally made from hydrophilic polymers and the hydroxy groups of these lenses attract and retain substantial amounts of water in the plastic which results in difficulties during cleaning and sterilization.

Furthermore, hydrophilic flexible contact lenses have a tendency to complex with and concentrate certain preservatives and disinfecting agents used in sterilizing conventional contact lenses. If these preservatives come into contact with the cornea they can cause severe irritation and burning.

Hydrogen peroxide systems, and particular a 3 % hydrogen peroxide solution, have emerged as the disinfectant of choice for all types of daily and extended wear hydrogen lenses. The primary reason for its increasing popularity is its rapid kill of microbial contaminants and its non-residual character. After hydrogen peroxide disinfects lenses, it can be converted into innocuous by-products which are compatible with ocular physiology. See Krezanoski et al., "Journal of the American Optometric Association", Vol. 59, Number 3, pages 193-197 (1988).

A great deal of patent literature is available concerning hydrogen peroxide contact lens disinfection systems. Reference is made in this respect to the following: Gaglia, Jr., U.S. Patent No. 3,912,451; LaRouzie et al., U.S. Patent No. 4,743,447; Davies et al., International Patent Publication WO 86/05695; Andermann et al., U.S. Patent No. 4,880,601; Giefer, U.S. Patent No. 4,585,488.

In general, the hydrogen peroxide systems involve a hydrogen peroxide-containing disinfecting solution into which the contact lenses to be disinfected are placed and allowed to remain for a required period of time. Nascent oxygen is released providing a germicidal effect. Following the requisite time period a purposeful inactivation of the hydrogen peroxide is conducted, for example, with a platinum catalyst. Following inactivation, the contact lens may be reinserted into the eye.

The hydrogen peroxide disinfecting solutions may be of the buffered or unbuffered type. As examples, AOSept® is a stabilized 3 % hydrogen peroxide solution made isotonic with sodium chloride and buffered to an approximate pH 6.9 with phosphates. On the other hand, LenSept® is a non-buffered 3 % hydrogen peroxide formulation.

The present invention is based upon the discovery that it is possible to improve the killing effectiveness and the rate of kill of both fungi and bacteria by modifying the buffered hydrogen peroxide disinfecting solution to incorporate therein an ocularly compatible surface active agent in a specified amount.

Thus, the invention is directed to a method for improving the efficacy of a buffered hydrogen peroxide formulation for disinfecting contact lenses which comprises incorporating into said formulation from about 0.1 % by weight to about 1.0 % by weight of the formulation of at least one ocularly compatible surface active agent which improves the killing efficacy of said buffered hydrogen peroxide formulation against fungal spores and staphylococcal strains.

In addition the invention is directed to a buffered hydrogen peroxide formulation for disinfecting contact lenses which contains
(a) from about 0.1 % by weight to about 1.0 % by weight of the formulation of a mixture of at least two ocularly compatible surface active agents or
(b) from about 0.1 % by weight to about 1.0 % by weight of the formulation of an ocularly compatible surface active agent selected from poloxamine 701, 702, 901, 1101, 1102, 1301, 1302, 1501 and 1502 surfactants, poloxamer surfactants, polysorbate surfactants, tyloxapol detergent, Miranol® amphoteric surfactants which are derived from coconut imidazole, Varonic® LI surfactants which are ethoxylated glyceryl monococoates and Varsulf® anionic sulfosuccinates, which ocularly compatible surface active agent or which mixture of ocularly compatible surface active agents improves the killing efficacy of said buffered hydrogen peroxide formulation.

Gaglia, Jr., U.S. Patent No. 3,912,451 discloses buffered hydrogen peroxide solutions of the type to which the present invention can be applied and for which improvement can be achieved.

With most 3 % hydrogen peroxide formulations, there are some viable organisms remaining even after a 30 minute soaking time of the contact lens. However, with the improvement of this invention, it is possible to provide a formulation in which there are no viable organisms after a 30 minute soaking time. With the current peroxide formulations there is a "lag" time between the time when the organisms are placed in the peroxide and the time when actual killing occurs. With the addition of the surface active agent, this "lag" time is reduced significantly and the rate of kill is enhanced.

In the literature it has been indicated that hydrogen peroxide formulations are not effective for contact lenses without a full-strength soaking for a minimum of 55 minutes. With this invention, however, it is possible to reduce considerably the disinfection time and obtain more complete disinfection.

The hydrogen peroxide formulations to which the surface active agent is added are of the buffered type. In general, in formulating such solutions a 3 % by weight hydrogen peroxide solution is formulated to contain 0.85 % by weight soldium chloride. Upon the ultimate conversion of the H₂O₂ to water and oxygen by catalyst this will yield an approximately isotonic saline solution as the final product. With the sodium chloride there is added to the H₂O₂ solution the necessary buffer system to buffer the 0.85 % aqueous sodium chloride solution to the desired pH of about 6.9 to 7.1.

The buffer system may comprise a suitable combination of monobasic sodium phosphate and dibasic sodium phosphate. Other satisfactory buffer systems may be employed which give substantially equivalent results in buffering to a pH of 6.9 to 7. 1. As examples there can be mentioned tartrate, succinate and glycine buffers or the MacIlvaine phosphatecitrate buffer as described in J. Biol. Chem., 183 (1921).

To the solution can also be added a hydrogen peroxide stabilizer such as sodium stannate or sodium nitrate. Such stabilizers are known in the art. In addition, as the stabilizer there can be employed diethylene triamine penta (methylenephosphonic acid) or a physiologically compatible salt thereof (see U.S. Patent No. 4,889,689).

As an example of the buffered type hydrogen peroxide formulation, there can be mentioned the commercially available AOSept® formulation.

In the broadest sense the surface active agents which can be used are those which are ocularly compatible and which function in the buffered hydrogen peroxide formulations to improve the killing effectiveness of such formulations. Preferably the surface active agent is a nonionic surface active agent. As specific examples of the surface active agents the following may be mentioned:

Polyethylene-polyoxypropylene substituted ethylenediamine nonionic surfactants which are more commonly known as "Tetronic®" type surfactants. These are also generally known by the generic name "poloxamine" and are commercially available from BASF-Wyandotte Corp.

Polyoxyethylene-polyoxypropylene nonionic surfactants sold under the trademark "Pluronic®" by BASF-Wyandotte Corp. These surfactants are generally known as "poloxamers " and typically have a molecular weight of between about 2,000 and about 5,000.

Polysorbates, such as "Tween® " surfactants (ICI Americas) including, for example polysorbate 20, 40, 60, 65, 80 and 85.

Tyloxapol, such as "Superinone® " detergent (Winthrop).

Miranol® amphoteric surfactants from Miranol Inc. such as "Miranol®" 2MCA which is lauryl sulfate salt of an amphoteric surfactant derived from coconut imidazoline.

Varonic® LI surfactants such as Varonic® LI-63 and LI-67 which are ethoxylated glyceryl monococoates. These are commercially available from Sherex Chemical Company, Inc..

Varsulf anionic sulfosuccinates such as Varsulf SBFA-30 and Varsulf SBL-203. These are commercially available from Sherex Chemical Company, Inc..

The foregoing are merely illustrative and not exhaustive of the surface active agents which can be employed in the invention.

One skilled in the art will be able to readily determine whether a given surface active agent or mixture of such agents can be employed. First, it is necessary to determine that such agent or mixture of agents is ocularly compatible. Tests for determining such compatibility are well known in the art. Such agent or mixture must not cause irritation or damage to the eye of a contact lens wearer. Secondly, the agent or mixture must be one which improves the killing efficacy of the buffered H₂O₂ formulation. Standard challenge organisms are known against which the buffered H₂O₂ formulation alone and that formulation containing the surface active agent or mixture can be compared. It is not, of course, essential that the agent or mixture of agents improve the efficacy against every challenge organism, it being most important that the overall spectrum of activity be improved.

It is also to be noted that these surface active agents can be employed alone or in combination. Preferred is e.g. a mixture of a poloxamine type and a poloxamer type surface active agent. Also preferred is a mixture of a poloxamine type and a polysorbate type surface active agent.

The surface active agent is added to the hydrogen peroxide formulation in an amount from about 0.1 % to about 1.0 % by weight of the formulation, preferably from about 0.1 % to about 0.6 % by weight of the formulation and more preferred from about 0.1 to about 0.4 % by weight of the formulation.

Particularly suitable for use in the invention are the nonionic poloxamine and poloxamer type surfactants as disclosed in European Patent Application EP-A2-439,429. These materials offer the further advantage of providing conditioning properties for the contact lenses by rendering the contact lens surface more wettable so that proteins, lipids and other tear film substituents do not adhere to and form deposits on the lens surface. Some relevant parts of EP-A2-439,429 are repeated hereinafter:
"The present invention relates to a conditioning solution for contact lenses which comprises an effective amount of a polyoxyethylene-polyoxypropylene substituted ethylenediamine nonionic surfactant having a hydrophile-lipophile balance of seven or below. These surfactants preferably have a molecular weight of between about 3,600 and about 9,000. Such surfactants are typically known generally as "Poloxamine", and sold under the trademark Tetronics® (BASF-Wyandotte Corp.). Preferred are those solutions wherein said polyoxyethylene-polyoxypropylene substituted ethylenediamine nonionic surfactant has a polyoxyethylene concentration between about 10 % and about 20 % by weight.

The solution may also have an effective amount of a polyoxyethylene-polyoxypropylene nonionic surfactant having a hydrophile-lipophile balance of seven or below and a polyoxyethylene concentration of less than about 20 % by weight. Such surfactants are generally known as "Poloxamers" and sold under the trademark Pluronics® (BASF-Wyandotte Corp.). They typically have a molecular weight of between about 2,000 and about 5,000.

The polyoxyethylene-polyoxypropylene substituted ethylenediamine nonionic surfactant and the polyoxyethylene-polyoxypropylene nonionic surfactant are both surface active agents which have as low a hydrophile-lipophile balance (HLB) as will be tolerated in the formulations. The low HLB values in the surface active agent indicate a high affinity for hydrophobic (lipophilic) surfaces. These surfaces active agents strongly adhere to those hydrophobic regions of the contact lens and render them hydrophilic. This adherence forms a "barrier" to potential absorbance, and keeps them from the surface of the lens. Furthermore, this increase in hydrophilicity simultaneously decreases the thermodynamic having force for protein and lipid absorption, thereby retarding tear film deposits.

As mentioned hereinbefore the poloxamine type surfactants are more commonly known as Tetronic® type surfactants. The Tetronic® type surfactant is a tetrafunctional block copolymer derived from the sequential addition of propylene oxide and ethylene oxide to ethylenediamine, and is represented by the following structure: 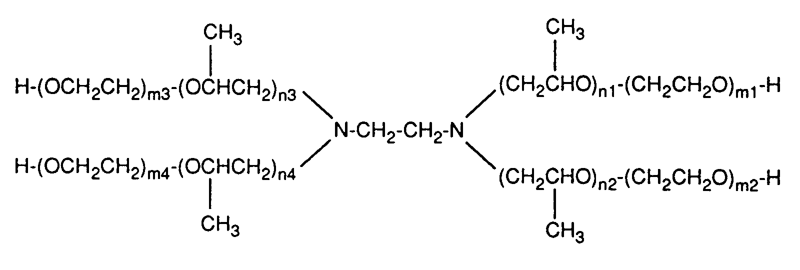 wherein n1 to n4 and m1 to m4 are numbers being a function of the desired molecular weight and the ratio of ethyleneoxy groups to propyleneoxy groups. The preparation thereof can be found in U.S. Patent No. 2,979,528, which is incorporated herein by reference. For convenience purposes, these nonionic surfactants will be identified generally as Tetronic®, with a numeral suffix to identify a particular grade of material as available from BASF-Wyandotte Corp.

It has been surprisingly discovered that only tetronics having a hydrophile-lipophile balance of seven or below are suitable for use in the conditioning solution of the present invention. Such tetronics typically have the molecular weight of between about 3,600 and about 9,000 and include Tetronic 701; 702; 901; 1101; 1102; 1301; 1302; 1501; and 1502 from BASF-Wyandotte.

The poloxamer type surfactants comprise a series of closely related block polymers that may generally be classified as polyoxyethylene-polyoxypropylene condensates terminating in hydroxyl groups. They are formed by the condensation of propylene oxide onto a propylene glyconucleus followed by the condensation of ethylene oxide onto both ends of polyoxypropylene base. The polyoxyethyl hydrophilic groups on the ends of the molecule are controlling length to constitute anywhere from 10 % to 80 % by weight of the final molecule. The structure of the polyethylene-polyoxypropylene nonionic surfactant is preferably as follows: 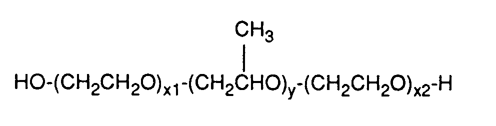 wherein x1, x2 and y are numbers being a function of the desired molecular weight and the ratio of ethyleneoxy groups to propyleneoxy groups. For convenience purposes, these nonionic surfactants will be referred to herein as Pluronic® generally, with a numerical suffix to identify a particular grade of material as available from BASF-Wyandotte Corp.

It has further been discovered that the polyoxyethylene-polyoxypropylene nonionic surfactant useful in the present invention must have a HLB of seven or below and a polyoxyethylene concentration of less than about 20 % by weight. Such preferred surfactants include Pluronic L61, L81, L101 and L121 from BASF-Wyandotte Corp. These polyoxyethylene-polyoxypropylene nonionic surfactants have a molecular weight of between about 2,000 and about 5,000."

Also particularly suitable for use is a mixture of a poloxamine, e.g. poloxamine 1302 (Tetronic®), and a polysorbate, e.g. polysorbate 80 (Tween® 80), in amounts of about 0.1 % by weight and about 0.4 % by weight respectively.

In order to exemplify the invention, compositions were compared for their relative disinfection capacity in respect to certain challenge microorganisms. The organisms tested are the FDA challenge organisms for disinfection solutions. The time required to kill 50 % of the colony forming units (CFU) in solution was designated as the D-value. The initial solution employed was a commercially available AOSept® solution which is a 3 % buffered hydrogen peroxide solution also containing a hydrogen peroxide stabilizer. The solution of the present invention for comparison is prepared by adding to the commercially available AOSept® initial solution, a mixture of polyoxamine 1302 (Tetronic®) and polysorbate 80 (Tween® 80) in amounts sufficient to constitute 0.1 % and 0.4 % by weight respectively of the formulation. The results are shown in the table below:

| | D-VALUE (MINUTES) | |
|---|---|---|
| Organism | AOSept® | Composition of the Invention |
| Pseudomonas aeruginosa | 0.54 | 0.53 |
| Staphylococcus epidermidis | 3.4 | 1.7 |
| Serratia marcescens | 2.4 | 3.5 |
| Candida albicans | 10 | 5.7 |
| Aspergillus fumigatus | 12.3 | 7.1 |

From the results it can be seen that the modified AOSept® formulation of the invention is particularly effective against fungal spores and staphylococcal strains.

Further tests of the inventors have revealed that the improvement of the invention is applicable to only buffered hydrogen peroxide formulation and not to unbuffered solutions.

For purposes of this test Aspergillus fumigatus was chosen as the test organism. Two representative hydrogen peroxide formulations were used. AOSept®, as previously indicated, is the commercially available 3 % stabilized hydrogen peroxide formulation that is buffered. LenSept® is a commercially available stabilized hydrogen peroxide formulation which is not buffered. These solutions were compared for their killing efficacy as the controls. To each of these control formulations were added specified amounts of various surfactants and the killing efficacy of the resultant solutions was determined. The test results are presented hereinafter. In these tests the D-value is the time necessary to kill 90 % of the active species.

### First Control: The D-Value for Lensept® is 8.5 minutes.

The D-Value for Lensept® comprising 0.1 % Tetronic 1302 is 10.8 minutes.
The D-Value for Lensept® comprising 0.4 % Tween 80 is 9.1 minutes.
The D-Value for Lensept® comprising 0.4 % Tyloxapol is 7.9 minutes.
The D-Value for Lensept® comprising 0.4 % Pluronic F 127 is 7.6 minutes.
The D-Value for Lensept® comprising 0.4 % Miranol 2MCA is 8.7 minutes.
The D-Value for Lensept® comprising 0.4 % Varsulf 30 - 40 % is 9.4 minutes.

### Second Control: The D-Value for AOSept® is 12.3 minutes.

The D-Value for AOSept® comprising 0.1 % Tetronic 1302 is 6.6 minutes.
The D-Value for AOSept® comprising 0.4 % Tween 80 is 7.7 minutes.
The D-Value for AOSept® comprising 0.4 % Tyloxapol is 8 minutes.
The D-Value for AOSept® comprising 0.4 % Pluronic F 127 is 7 minutes.
The D-Value for AOSept® comprising 0.4 % Miranol 2MCA is 4 minutes.
The D-Value for AOSept® comprising 0.4 % Varsulf 30 - 40 % is 6.9 minutes.

From the above data it can be seen that in the case of the addition of the surfactants to the AOSept® formulation, the killing efficacy was significantly increased in each case compared with the efficacy of the AOSept® alone. However, with the unbuffered LenSept® formulation, the addition of the surfactants either had an adverse affect on the killing efficacy (the time increased) or (in two cases) had a slight affect in improving it.

## Claims (Claims for the following Contracting State(s): AT, BE, CH, LI, DE, DK, FR, GB, GR, IT, LU, NL, PT, SE)

1. A method for improving the efficacy of a buffered hydrogen peroxide formulation for disinfecting contact lenses which comprises incorporating into said formulation from 0.1 % by weight to 1.0 % by weight of the formulation of at least one ocularly compatible surface active agent which improves the killing efficacy of said buffered hydrogen peroxide formulation against fungal spores and staphylococcal strains.

2. A method according to claim 1, wherein the hydrogen peroxide solution is buffered to a pH of 6.9 to 7.1.

3. A method according to claim 1, wherein the surface active agent is a nonionic surface active agent.

4. A method according to claim 3, wherein the surface active agent is a poloxamine.

5. A method according to claim 1, wherein a mixture of two surface active agents is employed.

6. A method according to claim 5, wherein a mixture of a poloxamine type and a poloxamer type surface active agent is employed.

7. A method according to claim 5, wherein a mixture of about 0.1 % by weight of the formulation of a poloxamine and about 0.4 % by weight of the formulation of a polysorbate is employed.

8. A buffered hydrogen peroxide formulation for disinfecting contact lenses which contains
(a) from 0.1 % by weight to 1.0 % by weight of the formulation of a mixture of at least two ocularly compatible surface active agents or
(b) from 0.1 % by weight to 1.0% by weight of the formulation of an ocularly compatible surface active agent selected from
poloxamine 701, 702, 901, 1101, 1102, 1301, 1302, 1501 and 1502 surfactants,
poloxamer surfactants,
polysorbate surfactants,
tyloxapol detergent,
Miranol® amphoteric surfactants which are derived from coconut imidazoline,
Varonic® LI surfactants which are ethoxylated glyceryl monococoates and
Varsulf® anionic sulfosuccinates,
which ocularly compatible surface active agent or which mixture of ocularly compatible surface active agents improves the killing efficacy of said buffered hydrogen peroxide formulation.

9. A formulation according to claim 8, wherein the mixture of surface active agents (a) comprises a nonionic surface active agent.

10. A formulation according to claim 9, wherein one of the surface active agents is a poloxamine.

11. A formulation according to claim 8, wherein a mixture of two surface active agents is employed.

12. A formulation according to claim 11, wherein a mixture of a poloxamine type and a poloxamer type surface active agent is employed.

13. A formulation according to claim 11, wherein a mixture of about 0.1 % by weight of the formulation of a poloxamine and about 0.4 % by weight of the formulation of a polysorbate is employed.

14. The use of a formulation according to claim 8 for the disinfection of a contact lens.

15. The use of a buffered hydrogen peroxide formulation comprising from 0.1 % by weight to 1.0 % by weight of the formulation of at least one ocularly compatible surface active agent which improves the killing efficacy of said buffered hydrogen peroxide formulation for the non-electrochemical disinfection of a contact lens.

## Claims (Claims for the following Contracting State(s): ES)

1. A method for improving the efficacy of a buffered hydrogen peroxide formulation for disinfecting contact lenses which comprises incorporating into said formulation from 0.1% by weight to 1.0% by weight of the formulation of at least one ocularly compatible surface active agent which improves the killing efficacy of said buffered hydrogen peroxide formulation against fungal spores and staphylococcal strains.

2. A method according to claim 1, wherein the hydrogen peroxide solution is buffered to a pH of 6.9 to 7.1.

3. A method according to claim 1, wherein the surface active agent is a nonionic surface active agent.

4. A method according to claim 3, wherein the surface active agent is a poloxamine.

5. A method according to claim 1, wherein a mixture of two surface active agents is employed.

6. A method according to claim 5, wherein a mixture of a poloxamine type and a poloxamer type surface active agent is employed.

7. A method according to claim 5, wherein a mixture of about 0.1 % by weight of the formulation of a poloxamine and about 0.4 % by weight of the formulation of a polysorbate is employed.

8. A process for manufacture of a buffered hydrogen peroxide formulation for disinfecting contact lenses which comprises adding to said formulation
(a) from 0.1 % by weight to 1.0% by weight of the formulation of a mixture of at least two ocularly compatible surface active agents or
(b) from 0.1 % by weight to 1.0 % by weight of the formulation of an ocularly compatible surface active agent selected from
poloxamine 701, 702, 901, 1101, 1102, 1301, 1302, 1501 and 1502 surfactants,
poloxamer surfactants,
polysorbate surfactants,
tyloxapol detergent,
Miranol® amphoteric surfactants which are derived from coconut imidazoline,
Varonic® LI surfactants which are ethoxylated glyceryl monococoates and
Varsulf® anionic sulfosuccinates,
which ocularly compatible surface active agent or which mixture of ocularly compatible surface active agents improves the killing efficacy of said buffered hydrogen peroxide formulation.

9. A process according to claim 8, wherein the mixture of surface active agents (a) comprises a nonionic surface active agent.

10. A process according to claim 9, wherein one of the surface active agents is a poloxamine.

11. A process according to claim 8, wherein a mixture of two surface active agents is employed.

12. A process according to claim 11, wherein a mixture of a poloxamine type and a poloxamer type surface active agent is employed.

13. A process according to claim 11, wherein a mixture of about 0.1 % by weight of the formulation of a poloxamine and about 0.4 % by weight of the formulation of a polysorbate is employed.

14. The use of a formulation according to claim 8 for the disinfection of a contact lens.

15. The use of a buffered hydrogen peroxide formulation comprising from 0.1 % by weight to 1.0 % by weight of the formulation of at least one ocularly compatible surface active agent which improves the killing efficacy of said buffered hydrogen peroxide formulation for the non-electrochemical disinfection of a contact lens.

## Patentansprüche (Patentansprüche für folgende(n) Vertragsstaat(en): AT, BE, CH, LI, DE, DK, ER, GB, GR, IT, LU, NL, PT, SE)

1. Verfahren zur Verbesserung der Wirksamkeit von gepufferter Wasserstoffperoxidformulierung zum Desinfizieren von Kontaktlinsen, welches das Einarbeiten von 0,1 Gewichtsprozent bis 1,0 Gewichtsprozent der Formulierung von mindestens einem augenverträglichen Tensid, das den Abtötungswirkungsgrad der gepufferten Wasserstoffperoxidformulierung gegen Pilzsporen und Staphylokokkenstämme verbessert, in die Formulierung umfaßt.

2. Verfahren nach Anspruch 1, wobei die Wasserstoffperoxidlösung auf einen pH-Wert von 6,9 bis 7,1 gepuffert wird.

3. Verfahren nach Anspruch 1, wobei das Tensid ein nichtionisches Tensid ist.

4. Verfahren nach Anspruch 3, wobei das Tensid ein Poloxamin ist.

5. Verfahren nach Anspruch 1, wobei ein Gemisch von zwei Tensiden angewendet wird.

6. Verfahren nach Anspruch 5, wobei ein Gemisch eines Tensids vom Poloxamintyp und eines Tensids vom Poloxamertyp angewendet wird.

7. Verfahren nach Anspruch 5, wobei ein Gemisch von etwa 0,1 Gewichtsprozent eines Poloxamins und etwa 0,4 Gewichtsprozent eines Polysorbats, bezogen auf die Formulierung, angewendet wird.

8. Gepufferte Wasserstoffperoxidformulierung zum Desinfizieren von Kontaktlinsen, die
(a) 0,1 Gewichtsprozent bis 1,0 Gewichtsprozent der Formulierung eines Gemisches von mindestens zwei augenverträglichen Tensiden oder
(b) 0,1 Gewichtsprozent bis 1,0 Gewichtsprozent der Formulierung eines augenverträglichen Tensids, ausgewählt aus
Poloxamin 701, 702, 901, 1101, 1102, 1301, 1302, 1501 und 1502 Tensiden,
Poloxamertensiden,
Polysorbattensiden,
Tyloxapoldetergent,
Miranol® amphoteren Tensiden, die von Kokosnußimidazolin abgeleitet sind,
Varonic® LI-Tensiden, die ethoxylierte Glycerylmonococoate darstellen und
Varsulf® anionischen Sulfosuccinaten,
enthält, wobei das augenverträgliche Tensid oder das Gemisch von augenverträglichen Tensiden den Abtötungswirkungsgrad der gepufferten Wasserstoffperoxidformulierung verbessert.

9. Formulierung nach Anspruch 8, wobei das Gemisch von Tensiden (a) ein nichtionisches Tensid umfaßt.

10. Formulierung nach Anspruch 9, wobei eines der Tenside ein Poloxamin darstellt.

11. Formulierung nach Anspruch 8, wobei ein Gemisch von zwei Tensiden angewendet wird.

12. Formulierung nach Anspruch 11, wobei ein Gemisch eines Tensids vom Poloxamintyp und eines Tensids vom Poloxamertyp angewendet wird.

13. Formulierung nach Anspruch 11, wobei ein Gemisch von etwa 0,1 Gewichtsprozent eines Poloxamins und etwa 0,4 Gewichtsprozent eines Polysorbats, bezogen auf die Formulierung, angewendet wird.

14. Verwendung einer Formulierung nach Anspruch 8 zur Desinfektion einer Kontaktlinse.

15. Verwendung einer gepufferten Wasserstoffperoxidformulierung, umfassend 0,1 Gewichtsprozent bis 1,0 Gewichtsprozent der Formulierung von mindestens einem augenverträglichen Tensid, das den Abtötungswirkungsgrad der gepufferten Wasserstoffperoxidformulierung zur nicht elektrochemischen Desinfektion einer Kontaktlinse verbessert.

## Patentansprüche (Patentansprüche für folgende(n) Vertragsstaat(en): ES)

1. Verfahren zur Verbesserung der Wirksamkeit von gepufferter Wasserstoffperoxidformulierung zum Desinfizieren von Kontaktlinsen, das Einarbeiten von 0,1 Gewichtsprozent bis 1,0 Gewichtsprozent der Formulierung von mindestens einem augenverträglichen Tensid, das den Abtötungswirkungsgrad der gepufferten Wasserstoffperoxidformulierung gegen Pilzsporen und Staphylokokkenstämme verbessert, in die Formulierung umfaßt.

2. Verfahren nach Anspruch 1, wobei die Wasserstoffperoxidlösung auf einen pH-Wert von 6,9 bis 7,1 gepuffert wird.

3. Verfahren nach Anspruch 1, wobei das Tensid ein nichtionisches Tensid ist.

4. Verfahren nach Anspruch 3, wobei das Tensid ein Poloxamin ist.

5. Verfahren nach Anspruch 1, wobei ein Gemisch von zwei Tensiden angewendet wird.

6. Verfahren nach Anspruch 5, wobei ein Gemisch eines Tensids vom Poloxamintyp und eines Tensids vom Poloxamertyp angewendet wird.

7. Verfahren nach Anspruch 5, wobei ein Gemisch von etwa 0,1 Gewichtsprozent eines Poloxamins und etwa 0,4 Gewichtsprozent eines Polysorbats, bezogen auf die Formulierung, angewendet wird.

8. Verfahren zur Herstellung einer gepufferten Wasserstoffperoxidformulierung zum Desinfizieren von Kontaktlinsen, umfassend die Zugabe zu der Formulierung von
(a) 0,1 Gewichtsprozent bis 1,0 Gewichtsprozent der Formulierung eines Gemisches von mindestens zwei augenverträglichen Tensiden oder
(b) 0,1 Gewichtsprozent bis 1,0 Gewichtsprozent der Formulierung eines augenverträglichen Tensids, ausgewählt aus
Poloxamin 701, 702, 901, 1101, 1102, 1301, 1302, 1501 und 1502 Tensiden,
Poloxamertensiden,
Polysorbattensiden,
Tyloxapoldetergent,
Miranol® amphoteren Tensiden, die von Kokosnußimidazolin abgeleitet sind,
Varonic® LI-Tensiden, die ethoxylierte Glycerylmonococoate darstellen und
Varsulf® anionischen Sulfosuccinaten,
enthält, wobei das augenverträgliche Tensid oder das Gemisch von augenverträglichen Tensiden den Abtötungswirkungsgrad der gepufferten Wasserstoffperoxidformulierung verbessert.

9. Verfahren nach Anspruch 8, wobei das Gemisch von Tensiden (a) ein nichtionisches Tensid umfaßt.

10. Verfahren nach Anspruch 9, wobei eines der Tenside ein Poloxamin darstellt.

11. Verfahren nach Anspruch 8, wobei ein Gemisch von zwei Tensiden angewendet wird.

12. Verfahren nach Anspruch 11, wobei ein Gemisch eines Tensids vom Poloxamintyp und eines Tensids vom Poloxamertyp angewendet wird.

13. Verfahren nach Anspruch 11, wobei ein Gemisch von etwa 0,1 Gewichtsprozent eines Poloxamins und etwa 0,4 Gewichtsprozent eines Polysorbats, bezogen auf die Formulierung, angewendet wird.

14. Verwendung einer Formulierung nach Anspruch 8 zur Desinfektion einer Kontaktlinse.

15. Verwendung einer gepufferten Wasserstoffperoxidformulierung, umfassend 0,1 Gewichtsprozent bis 1,0 Gewichtsprozent der Formulierung von mindestens einem augenverträglichen Tensid, das den Abtötungswirkungsgrad der gepufferten Wasserstoffperoxidformulierung zur nicht elektrochemischen Desinfektion einer Kontaktlinse verbessert.

## Revendications (Revendications pour l'(les) Etat(s) contractant(s) suivant(s): AT, BE, CH, LI, DE, DK, FR, GB, GR, IT, LU, NL, PT, SE)

1. Une méthode pour améliorer l'efficacité d'une formulation tamponnée de peroxyde d'hydrogène pour décontaminer les lentilles de contact, caractérisée en ce qu'on incorpore dans ladite formulation de 0,1 % en poids à 1,0% en poids de la formulation d'au moins un agent actif en surface compatible du point de vue oculaire qui améliore l'efficacité de destruction de ladite formulation tamponnée de peroxyde d'hydrogène contre les spores fongiques et les souches de staphylocoques.

2. Une méthode selon la revendication 1, caractérisée en ce que la solution de peroxyde d'hydrogène est tamponnée à un pH de 6,9 à 7,1.

3. Une méthode selon la revendication 1, caractérisée en ce que l'agent actif en surface est un agent actif en surface non ionique.

4. Une méthode selon la revendication 3, caractérisée en ce que l'agent actif en surface est une poloxamine.

5. Une méthode selon la revendication 1, caractérisée en ce qu'on utilise un mélange de 2 agents actifs en surface.

6. Une méthode selon la revendication 5, caractérisée en ce qu'on utilise un mélange d'un agent actif en surface du type poloxamine et d'un agent actif en surface du type poloxamer.

7. Une méthode selon la revendication 5, caractérisée en ce qu'on utilise un mélange d'environ 0,1% en poids de la formulation d'une poloxamine et environ 0,4% en poids de la formulation d'un polysorbate.

8. Une formulation tamponnée de peroxyde d'hydrogène pour décontaminer les lentilles de contact qui contient
(a) de 0,1% en poids à 1,0% en poids de la formulation d'un mélange d'au moins deux agents actifs en surface compatibles du point de vue oculaire ou bien
(b) de 0,1% en poids à 1,0% en poids de la formulation d'un agent actif en surface compatible du point de vue oculaire choisi parmi
les tensio-actifs du type poloxamine 701, 702, 901, 1101, 1102, 1301, 1302, 1501 et 1502,
les tensio-actifs du type poloxamer,
les tensio-actifs du type polysorbate,
le détergent tyloxapol,
les tensio-actifs amphotères de la marque Miranol® qui sont dérivés de l'imidazoline de coco,
les tensio-actifs de la marque Varonic® LI qui sont des monococoates de glycéryle éthoxylés et
les sulfosuccinates anioniques de la marque Varsulf®,
ledit agent actif en surface compatible du point de vue oculaire ou ledit mélange d'agents actifs en surface compatibles du point de vue oculaire améliore l'efficacité de destruction de ladite formulation tamponnée de peroxyde d'hydrogène.

9. Une formulation selon la revendication 8, caractérisée en ce que le mélange d'agents actifs en surface (a) comprend un agent actif en surface non ionique.

10. Une formulation selon la revendication 9, caractérisée en ce qu'un des agents actifs en surface est une poloxamine.

11. Une formulation selon la revendication 8, caractérisée en ce qu'on utilise un mélange de 2 agents actifs en surface.

12. Une formulation selon la revendication 11, caractérisée en ce qu'on utilise un mélange d'un agent actif en surface du type poloxamine et d'un agent actif en surface du type poloxamer.

13. Une formulation selon la revendication 11, caractérisée en ce qu'on utilise un mélange d'environ 0,1% en poids de la formulation d'une poloxamine et environ 0,4% en poids de la formulation d'un polysorbate.

14. L'utilisation d'une formulation selon la revendication 8, pour décontaminer une lentille de contact.

15. L'utilisation d'une formulation tamponnée de peroxyde d'hydrogène comprenant de 0,1% en poids à 1,0% en poids de la formulation d'au moins un agent actif en surface compatible du point de vue oculaire qui améliore l'efficacité de destruction de ladite formulation tamponnée de peroxyde d'hydrogène pour la décontamination non électrochimique d'une lentille de contact.

## Revendications (Revendications pour l'(les) Etat(s) contractant(s) suivant(s): ES)

1. Une méthode pour améliorer l'efficacité d'une formulation tamponnée de peroxyde d'hydrogène pour décontaminer les lentilles de contact, caractérisée en ce qu'on incorpore dans ladite formulation de 0,1 % en poids à 1,0% en poids de la formulation d'au moins un agent actif en surface compatible du point de vue oculaire qui améliore l'efficacité de destruction de ladite formulation tamponnée de peroxyde d'hydrogène contre les spores fongiques et les souches de staphylocoques.

2. Une méthode selon la revendication 1, caractérisée en ce que la solution de peroxyde d'hydrogène est tamponnée à un pH de 6,9 à 7,1.

3. Une méthode selon la revendication 1, caractérisée en ce que l'agent actif en surface est un agent actif en surface non ionique.

4. Une méthode selon la revendication 3, caractérisée en ce que l'agent actif en surface est une poloxamine.

5. Une méthode selon la revendication 1, caractérisée en ce qu'on utilise un mélange de 2 agents actifs en surface.

6. Une méthode selon la revendication 5, caractérisée en ce qu'on utilise un mélange d'un agent actif en surface du type poloxamine et d'un agent actif en surface du type poloxamer.

7. Une méthode selon la revendication 5, caractérisée en ce qu'on utilise un mélange d'environ 0,1% en poids de la formulation d'une poloxamine et environ 0,4% en poids de la formulation d'un polysorbate.

8. Un procédé de fabrication d'une formulation tamponnée de peroxyde d'hydrogène pour décontaminer les lentilles de contact, caractérisé en ce qu'on ajoute à ladite formulation
(a) de 0,1% en poids à 1,0% en poids de la formulation d'un mélange d'au moins deux agents actifs en surface compatibles du point de vue oculaire ou bien
(b) de 0,1% en poids à 1,0% en poids de la formulation d'un agent actif en surface compatible du point de vue oculaire choisi parmi
les tensio-actifs du type poloxamine 701, 702, 901, 1101, 1102, 1301, 1302, 1501 et 1502,
les tensio-actifs du type poloxamer,
les tensio-actifs du type polysorbate,
le détergent tyloxapol,
les tensio-actifs amphotères de la marque Miranol® qui sont dérivés de l'imidazoline de coco,
les tensio-actifs de la marque Varonic® LI qui sont des monococoates de glycéryle éthoxylés et
les sulfosuccinates anioniques de la marque Varsulf®,
ledit agent actif en surface compatible du point de vue oculaire ou ledit mélange d'agents actifs en surface compatibles du point de vue oculaire améliore l'efficacité de destruction de ladite formulation tamponnée de peroxyde d'hydrogène.

9. Un procédé selon la revendication 8, caractérisé en ce que le mélange d'agents actifs en surface (a) comprend un agent actif en surface non ionique.

10. Un procédé selon la revendication 9, caractérisé en ce qu'un des agents actifs en surface est une poloxamine.

11. Un procédé selon la revendication 8, caractérisé en ce qu'on utilise un mélange de 2 agents actifs en surface.

12. Un procédé selon la revendication 11, caractérisé en ce qu'on utilise un mélange d'un agent actif en surface du type poloxamine et d'un agent actif en surface du type poloxamer.

13. Un procédé selon la revendication 11, caractérisé en ce qu'on utilise un mélange d'environ 0,1% en poids de la formulation d'une poloxamine et environ 0,4% en poids de la formulation d'un polysorbate.

14. L'utilisation d'une formulation selon la revendication 8, pour décontaminer une lentille de contact.

15. L'utilisation d'une formulation tamponnée de peroxyde d'hydrogène comprenant de 0,1% en poids à 1,0% en poids de la formulation d'au moins un agent actif en surface compatible du point de vue oculaire qui améliore l'efficacité de destruction de ladite formulation tamponnée de peroxyde d'hydrogène pour la décontamination non électrochimique d'une lentille de contact.
